Europäisches Patentamt

European Patent Office

Office européen des brevets

(11) Publication number: 0 404 456 A2

# EUROPEAN PATENT APPLICATION

(21) Application number: 90306553.0

(22) Date of filing: 15.06.90

(51) Int. Cl.5: G01N 33/48, G01N 11/10

(30) Priority: 19.06.89 US 367853

(43) Date of publication of application:
27.12.90 Bulletin 90/52

(84) Designated Contracting States:
BE DE FR GB GR IT

(71) Applicant: HAEMOSCOPE CORPORATION
5836 Lincoln Avenue
Morton Grove, Illinois 60053(US)

(72) Inventor: Zuckerman, Leon
8942 Crawford
Skokie, Illinois 60076(US)

(74) Representative: Bayliss, Geoffrey Cyril et al
BOULT, WADE & TENNANT 27 Furnival Street
London EC4A 1PQ(GB)

(54) Disposable pin and cup with reuseable stem and collar for blood coagulation analyzer.

(57) An instrument (12) for measuring the coagulation characteristics of whole blood samples is provided with disposable parts for contacting the blood. The pin has a blood-compatible plastic sleeve (18) surrounding a metal stem (20) which weights the pin assembly so it sinks into the blood sample. The metal stem also engages into the pin suspension apparatus of the instrument. A blood-compatible plastic cup (14) receives the blood sample to be tested, and is surrounded by a metal collar (16) to transfer heat to the cup for the time required for a test. The plastic surfaces which contact the blood sample are slightly roughened, to a matte finish, to improve adhesion so as to approximate the results obtained with stainless steel pins and cups.

FIG.2

# DISPOSABLE PIN AND CUP WITH REUSEABLE STEM AND COLLAR FOR BLOOD COAGULATION ANA-LYZER

The present invention relates to blood coagulation test instruments.

Measurement of the ability of a patient's blood to coagulate in timely and effective fashion is crucial to certain surgical and medical procedures. Blood coagulation analyzer instruments have been known since Helmut Hartert developed them in Germany in the 1940's. A U.S. patent showing the background and continued development of the Hellige line of such instruments is no. 3,714,815.

In such instruments, a small stainless steel cup is pre-warmed to body temperature. A sample of whole blood is placed into the cup. A cylindric pin suspended on a thin wire is lowered into the cup and the blood sample, which then is covered with mineral oil. The cup is oscillated gently, over about 10 seconds, back and forth through a small angle, about 4 degrees, around its vertical axis. As the blood coagulates or clots, elements in the blood link the cup and the pin surfaces together, so the pin too begins to oscillate. The oscillations of the pin are detected by reflected light beams, magnetically, or otherwise. Ultimately the pin and the cup oscillate together, if the clot is strong and does not break up.

Delay in onset of the clot, weakness of the clot, or breaking up of the clot are shown clearly in a real-time plot of the movements of the pin. The plot may be expressed by mathematical factors characterizing its shape.

Blood may be contaminated with hepatitis virus, AIDS virus, and other infectious agents dangerous to the personnel operating the blood test equipment. Cleaning of the surfaces contacting the blood necessarily brings such personnel into contact with the blood on the surfaces, particularly where the blood has congealed. Cleaning and drying must nonetheless be thorough, and the surfaces must not be scratched, to ensure accuracy of subsequent test results.

Although the desireability of use of disposable blood coagulation test implements has been recognised generally, no practicable structures for disposable pins and/or cups for use in blood coagulation test equipment have been proposed. Merely replicating the conventional stainless steel pins and cups in plastic does not overcome problems such as actually sinking the suspended pin in the blood, avoiding breakage of the thin shaft of the pin where it is inserted into the suspension and measuring assembly, and transferring adequate heat to the blood in the cup.

A pin and a cup for blood coagulation test instruments have blood-compatible plastic such as Cyro-G20 with slightly matte or roughened surfaces to contact the blood to be tested. The cup is formed of such plastic, with an axially-symmetric inner or side wall surface in the area contacting the blood. Above the blood well the inner wall tapers sharply outwardly. A metal collar fits closely about the outside of the cup. The pin has a sleeve also formed of such plastic with an outer wall corresponding in size and shape to the inner wall of the cup, providing clearance for the blood. The sleeve thins in an upward area adjacent the tapered portion of the cup wall, and ends upwardly in a radially-extending head, to afford easier manipulation. The pin comprises also an inner metal stem having a lower end received snugly but removably in the plastic sleeve. An upper end of the stem is releasably engageable with the suspension and measuring apparatus of the test instrument. A central portion of the stem is enlarged to afford easier manipulation and to weight the pin so it will sink in the blood sample in the cup when suspended from a thin wire. The pin and cup of the invention are used in the same fashion and provide closely similar results as known stainless steel pins and cups, but after a test is conducted the used sleeve is quickly disconnected from the stem and discarded. The used cup is lifted from the metal collar and discarded. The stem and collar, never having touched blood, are easily and safely reused.

The following is a description of a specific embodiment of the invention reference being made to the accompanying drawings:

FIG. 1 is a general perspective view of the pin and cup of the invention in use in the test area of a known blood coagulation test instrument;

FIG. 2 is an exploded perspective view of the pin and cup of the invention;

FIG. 3 is a longitudinal view, partly in section on line 3-3 of Figure 1, through the cup and pin of the invention and the suspension apparatus, and showing a raised position of the pin in phantom;

FIG. 4 is an axial sectional view, on line 4-4 of Figure 3, through the cup and pin of the invention;

FIG. 5 is a longitudinal sectional view, on line 5-5 of Figure 4, through the sleeve and stem of the pin of the invention; and

FIG. 6 is an axial sectional view, on line 6-6 of Figure 5, through the sleeve of the invention.

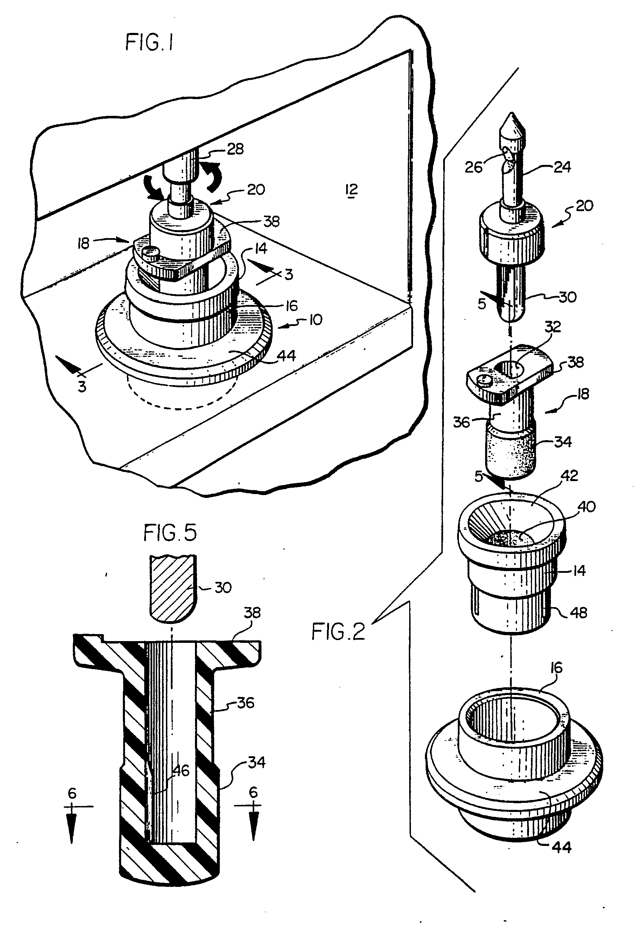

A disposable pin and cup assembly 10 according to one form of the invention is shown in Fig. 1 in use during a blood coagulation test in a test instrument 12 such as a Hellige THROMBELASTOGRAPH D instrument. The test instrument includes oscillation means, not shown, for holding and gently oscillating a cup 14 containing a blood sample to be tested through a small arc, about 4 degrees 45 minutes of arc, with each oscillation (back and forth) being completed in about 10 seconds. A metal collar 16 fits closely about the cup 14 and helps to maintain the desired temperature of the blood.

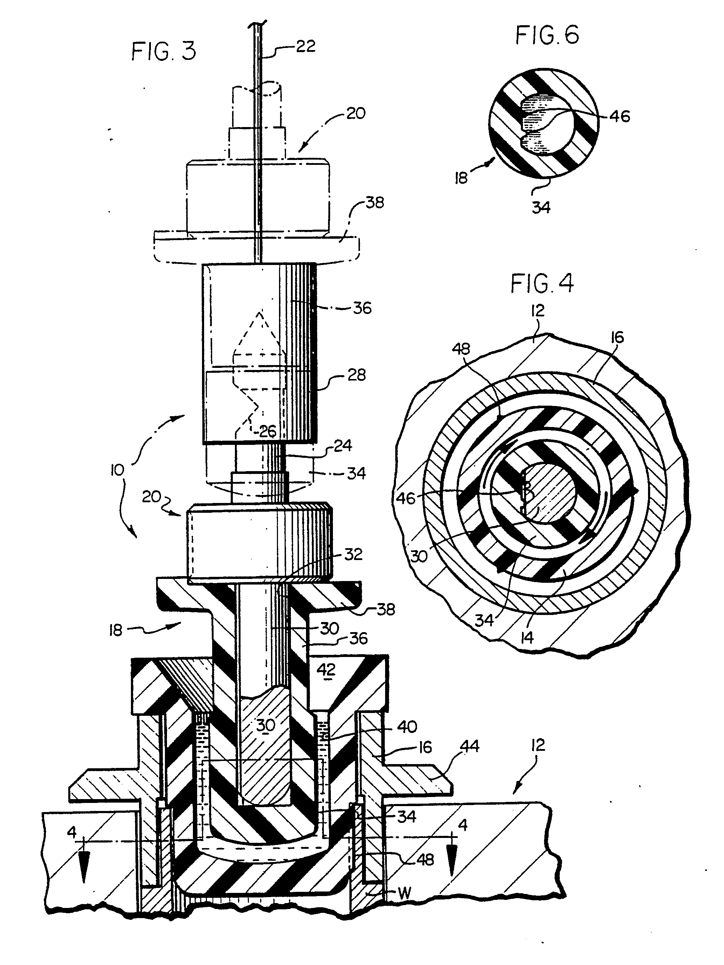

A pin comprising a plastic sleeve 18 and a metal stem 20 is suspended from a thin wire 22 in the instrument 12, as shown in Figure 3. The stem 20 is formed with an upper engagement portion 24 including an alignment notch 26, which removably engages into a receptacle 28 affixed to the wire 22. The stem also has a lower portion 30 which is removably inserted into a central axial opening 32 of the sleeve 18. A center portion of the stem 20 is enlarged as shown to add weight to the pin assembly to sink same into the blood, to allow easier manipulation, and to provide a stop surface for the sleeve 18.

The sleeve 18 is formed of a blood-compatible plastic such as Cyro-G20. It has an axially-symmetric, slightly roughened or matte-finish outer test surface 34 extending for a distance above its lowermost point as shown in Figures 2, 3, and 5, with a reduced-diameter portion 36 above that, and then a radially-extending head 38 at the top. The top of the head 38 contacts the enlarged center portion of the stem to ensure proper seating of the sleeve on the stem and to ease manipulation.

The cup 14 is also formed of a blood-compatible plastic such as Cyro-G20. The cup has an inner wall 40 corresponding in size and shape to the outer surface 34 of the sleeve 18. The inner wall 40 is also axially-symmetric, and extends a distance above its bottom about equal to that of the sleeve surface 34. It has a slightly roughened or matte finish. The inner surface of the cup 14 then tapers outwardly quickly near the top of the cup, as at surface 42, to accommodate excess oil or blood placed into the cup without affecting test results.

The blood-contacting surfaces 34 and 40 are formed with a slight roughness or matte finish, which is imparted by sand-blasting the corresponding surfaces of stainless steel forming or casting molds. Tested by ANSI No. B46.1 (1978), the sleeve surface 34 has a roughness of 4.0 to 9.9 microinches and the cup inner surface 40 has a roughness of 17.7 to 33.5 microinches in representative samples. The roughness enhances the adhesion of clots of blood to the plastic and provides results using the plastic parts to be closely similar to results obtained using conventional stainless steel pins and cups.

An outer wall of the cup 14 is formed to fit closely into the metal collar 16, but with some clearance. A lower part of the cup is snugly received in a well W, which is heated and which oscillates within the instrument 12, so the cup will oscillate with the collar in the test station of instrument 12. The collar 16 is formed of a heat-conductive metal such as aluminum, to conduct heat from the test station 12 through the plastic cup 14 to the blood in the cup throughout the test procedure. A wide flange 44 on the outside of the collar is slightly elevated above the surfaces of the test station 12, to facilitate removal of the collar and cup.

At least one internal surface of the sleeve 18 which engages a metal surface of the stem 20 is formed with integrally molded crush lines or projections 46 as shown in Figures 4, 5, and 6. These crush lines ensure the tightness of fit between the stem and the sleeve, to ensure reproducable test results. Similar projections 48 are provided on well W and the outside of the cup 14 to insure firm contact with the cup drive mechanism (not shown).

In use, the collar 16 and cup 14 are prewarmed to body temperature by test station area 12, which is also heated to maintain the blood sample's temperature during the 20-40 minute test. The collar 16 insures even heating of all of the cup 14. The stem 20 and sleeve 18 are assembled together by inserting the lower portion 30 of the stem into the central axial hollow 32 of the sleeve. The upper portion 24 of the stem is aligned and engaged at its notch 26 with the receptacle 28 of the test instrument 12. A sample of whole blood to be tested is placed in the plastic cup 14 surrounded by the metal collar 16 in the test station of the instrument 12. The wire 22 and pin assembly are then lowered into the test position, with the lower surface 34 of the sleeve 18 immersed in the blood within cup 14. A thin film of mineral oil is applied to the surface of the blood, about the pin, to reduce contact of the blood with air. The oscillation of well W is started, and recording of the oscillation of the pin assembly induced by clotting of the blood sample is shown or measured and recorded on an output device (not shown).

To remove the pin and cup from the test instrument 12, the pin is raised in the machine. The stem and sleeve are pulled downward from the receptacle and placed back into the cup. The whole pin, cup, and collar assembly is next lifted and removed from the test instrument. The stem and sleeve are separated by prying them apart between the enlarged stem center portion and the head of the sleeve, and the cup and sleeve are discarded. The stem and collar are then reused with new plastic parts.

Other forms of blood coagulation test equipment may be used with the disposable pin and cup of the invention, to good advantage. If the pin is rotated, or if other forms of measurement are used, the parts may be adapted accordingly and the same advantages obtained.

The present invention is disclosed in a preferred form as presently known and practiced. Other forms of the invention may readily be devised to vary and to improve the application of same in different environments or uses. The present invention is not solely defined or limited by what is specifically shown or described herein, but is indicated in the appended claims.

## Claims

1. For use in an instrument for measuring clotting characteristics of blood, the instrument having a cup with a substantially axially-symmetric inner wall for receiving and holding a sample of said blood, a pin having a corresponding outer wall receivable in said cup and said blood sample generally along an axis of said cup, holding means for holding said pin and cup in a desired spacial relation to one another, oscillation means for inducing oscillation of one of said cup and said pin generally about said axis, and measuring and output means for indicating the change in oscillation of at least one of said pin and cup caused by clotting of the blood, an improved pin comprising:
a sleeve forming said outer wall and being comprised of a plastic material and having a central axial opening from at least a top end of said sleeve; and
a durable stem having a first end thereof removably receivable in said opening of said sleeve, said stem being engageable at an opposite end thereof with said holding means of said instrument,
whereby the sleeve but not the stem is placed in contact with said blood sample for measuring the clotting characteristics thereof and the sleeve is separable from the stem and is economically disposable after use.

2. An improved pin as defined in Claim 1, wherein the stem is of a weight and density that the stem and sleeve do not float but sink in the blood sample in the cup without force being applied by said holding means.

3. An improved pin as defined in Claim 1, wherein the central axial opening of the sleeve is formed with deformable members therein for closely and tightly engaging a portion of the stem inserted thereinto.

4. An improved pin as defined in Claim 1, wherein the outside wall of the sleeve has a uniform outside diameter for an axial distance and the inside wall of the cup has a uniform inside diameter for at least said distance above a bottom of said cup, whereby any clotting of said sample of blood occurs between said walls.

5. An improved pin as defined in Claim 1, wherein the sleeve is formed with an enlarged, radially-extending head spaced above the uniform wall thereof.

6. An improved pin as defined in Claim 1, wherein the sleeve is formed of a blood-compatible plastic material, such as Cyro-G20.

7. An improved pin as defined in Claim 1, wherein the outer wall of the sleeve has a roughened surface, for enhancing binding of a blood clot to said plastic.

8. An improved pin as defined in Claim 7, wherein the surface has a roughness measured in a range of about 4 to 10 microinches under ANSI Standard No. B46.1 (1978).

9. A pin and cup assembly for use in an instrument for measuring coagulation of a blood sample, the pin and the cup being held in relative positions in said instrument for transfer of an oscillation of one to the other as the blood coagulates, and the instrument including detection and measurement means for indicating the time and strength of coagulation of the blood sample being tested, the assembly comprising:
a cup formed of a plastic material and having a substantially axially-symmetric inner wall for receiving and holding a sample of said blood;
a collar formed of a metal material and sized to fit closely about an outside portion of the cup, thereby to conduct heat to said cup during a coagulation test;
a sleeve forming an axially-symmetric outer wall receivable in said cup and in said blood sample and generally along the axis of said cup, said sleeve being comprised of a plastic material and having a central axial opening from a top end of said sleeve; and
a stem having a first end thereof removably receivable in said opening of said sleeve, said stem being engageable at an opposite end thereof with said instrument,
whereby the sample of blood contacts only the plastic cup and sleeve and not the collar and the stem, so that the cup and sleeve may economically be discarded after test of said sample and the collar and stem reused.

10. A pin and cup assembly as defined in Claim 9, wherein the collar is formed with 2 radially and outwardly-extending circumferential flanges.

11. A pin and cup assembly as defined in Claim 9, wherein the cup has a uniform inner diameter for an axial distance and then tapers outwardly at an upper end thereof, and wherein said sleeve has a uniform outer diameter extending for an axial distance less than that of said cup and then a lesser diameter beyond said distance.

12. A pin and cup assembly as defined in Claim 9, wherein the sleeve and stem together are of a weight and density that they sink rather than floating in blood in said cup.

13. A pin and cup assembly as defined in Claim 9, wherein the sleeve is formed with a head radially extending therefrom at a topmost portion thereof.

14. A pin and cup assembly as defined in Claim 9, wherein the sleeve and the cup are formed of blood-compatible materials, such as Cyro-G20, and the surfaces thereof which contact the blood are slightly rough.

15. A pin and cup assembly as defined in Claim 14, wherein said surfaces have a roughness measured under ANSI Standard No. B46.1 (1978) in a range of about 4 to 10 microinches on the sleeve and about 17 to 34 microinches on the cup.

16. A pin and cup for use in testing of blood for its coagulation characteristics, in an instrument which establishes relative movement between the pin and the cup and measures the linking between them as the blood coagulates, wherein:
said pin comprises a disposable sleeve fitted over a reusable metal stem;
the sleeve has an axially-symmetric outer surface formed of a blood-compatible plastic and an inner opening for releasably accepting said stem;
the stem has a first end releasably received in the sleeve and a second end releasably engageable with said instrument;
said cup has an axially-symmetric inner surface sized to accept therein said sleeve with a desired clearance to allow the blood to coagulate between them, the cup inner surface being formed of a blood-compatible plastic; and
said outer surface of said sleeve and said inner surface of said cup are slightly rough, to enhance binding of blood to said surface,
whereby after a blood coagulation test the blood-engaging sleeve and cup may be separated from reusable components and discarded and the reuseable stem used again with new sleeves and cups.

17. A pin and cup as defined in Claim 16, further comprising a reuseable metal collar sized to fit closely about an outside of said plastic cup and inside said instrument, whereby the collar transfers heat to said cup and blood therein.

18. A pin and cup as defined in Claim 16, wherein the stem further comprises a weight sufficient to cause the pin to sink in blood rather than float in same.

19. A pin and cup as defined in Claim 16, wherein the outer surface of said sleeve has a roughness measured under ANSI Standard No. B46.1 (1978) in a range of about 4 to 10 microinches and the inner surface of said cup has a roughness under said Standard in a range of about 17 to 34 microinches.

FIG.1

28
20
38
18
14
3
16
10
44
12
3

FIG.5

30
38
36
46
34
6
6

FIG.2

26
24
20
30
5
32
38
18
36
34
5
42
40
14
48
16
44

FIG. 3

FIG. 6

FIG. 4